# EUROPEAN PATENT SPECIFICATION

(11) **EP 3 158 082 B1**
(45) Date of publication and mention of the grant of the patent: **30.12.2020**
(21) Application number: 15730159.9
(22) Date of filing: 19.06.2015
(51) Int. Cl.: C12Q 1/68

(54) **RESTRICTION MEDIATED QUANTITATIVE POLYMERASE CHAIN REACTIONS**
EINSCHRÄNKUNGSVERMITTELTE QUANTITATIVE POLYMERASEKETTENREAKTIONEN
RÉACTIONS EN CHAÎNE PAR POLYMÉRASE QUANTITATIVES MÉDIÉES PAR RESTRICTION

(30) Priority: 19.06.2014 WO PCT/EP2014/062971
(43) Date of publication of application: 26.04.2017
(73) Proprietor: Multiplicom NV, 2845 Niel (BE)
(72) Inventor: DEL FAVERO, Jurgen, 2845 Niel (BE)
(74) Representative: Boult Wade Tennant LLP
(86) International application number: PCT/EP2015/063832
(87) International publication number: WO 2015/193483

(56) References cited:
- WO-A1-01/38570
- WO-A1-2010/139937
- WO-A1-2011/141738
- WO-A1-2012/024639
- WO-A2-2011/063388
- SOLINAS A ET AL: "Duplex Scorpion primers in SNP analysis and FRET applications", NUCLEIC ACIDS RESEARCH, INFORMATION RETRIEVAL LTD, vol. 29, no. 20, 15 October 2001 (2001-10-15), page E96, XP002318909, ISSN: 0305-1048, DOI: 10.1093/NAR/29.20.E96

## Description

The present invention relates to the technical field of nucleic acid amplification using a Polymerase Chain Reaction (PCR). Specifically, the present invention relates to Polymerase Chain Reaction (PCR) primers, Polymerase Chain Reaction (PCR) nucleic acid amplification mixtures and the use thereof in, quantitative Polymerase Chain Reactions (PCR) and especially quantitative Polymerase Chain Reaction (qPCR) allowing amplification of multiple amplicons in a single Polymerase Chain Reaction (PCR).

A quantitative polymerase chain reaction (qPCR), also designated as a real-time polymerase chain reaction, is a laboratory technique generally used in Molecular Biology based on the polymerase chain reaction (PCR).

A polymerase chain reaction (PCR) generally consists of series of repeated, for example 15 to 60 times, cycles of temperature changes. These temperature cycles generally comprise at least three stages: the first stage, at around 95 °C, allows for separation of the nucleic acid's double chain; the second stage, at a temperature of around 45 to 65°C, allows for binding of primers with a DNA template and the third stage, at between 68 to 75 °C, facilitates polymerization carried out by a DNA polymerase thereby providing amplification of a target nucleic acid sequence. The specific temperatures and the time intervals used in each cycle depend on a number of parameters, such as enzymes used to duplicate the target DNA, concentration of divalent ions and deoxyribonucleotides (dNTPs) and annealing temperatures of the primers used.

A quantitative polymerase chain reaction (qPCR) is generally used to amplify, and quantify, a targeted DNA molecule. For one, or more, specific nucleic acid sequences in a DNA sample, quantitative PCR enables both detection and quantification. The quantity can be either an absolute number of copies or a relative amount when the amplification products are normalized to DNA input or reference genes. Quantitative PCR is generally carried out in a thermal cycler with the capacity to illuminate each sample with a beam of light of a specified wavelength and to detect the fluorescence emitted by the excited chromo- or fluorophore. Solinas et al. (Nucleic Acids Research, 2001, Vol. 29, No. 20 e96) describes Scorpion primers for use in real-time PCR.

Multiplex polymerase chain reaction (Multiplex PCR) is a modification of polymerase chain reaction. A multiplex polymerase chain reaction uses two or more primer sets within a single PCR mixture to produce amplicons that are specific to different DNA sequences. By pursuing multiple targets at once, additional information may be gained from a single test run otherwise requiring several separate PCR reactions.

A combination of the above PCR assays, i.e. a quantitative polymerase chain reaction (qPCR) and multiplex polymerase chain reaction (multiplex PCR) would allow of real-time detection of multiple target sequences using a single amplification reaction thereby allowing, amongst others, efficient high through-put diagnosis on relatively small biological samples. However, using the presently known techniques, combining qPCR and multiplex PCR can only allow for the simultaneous amplification of a limited number of amplicons (up to 3 amplicons) in a single PCR reaction.

It is an object of the present invention, amongst other objects, to provide a combined use of a quantitative polymerase chain reaction (qPCR) and multiplex polymerase chain reaction (multiplex PCR) allowing for the simultaneous amplification of 4 or more amplicons such as more 10, more than 20, more than 40 or even more than 40 amplicons.

The above object of the present invention, amongst other objects, is met by the present invention which is defined in the claims. The present novel PCR assay is designated herein as RM-QPCR or Restriction Mediated Quantitative PCR.

The above object of the present invention, amongst other objects, is, according to a first aspect, met by Polymerase Chain Reaction (PCR) primer suitable for use in Restriction Mediated quantitative PCR (RM-qPCR) nucleic acid amplification reactions, said primer has the following general structure: 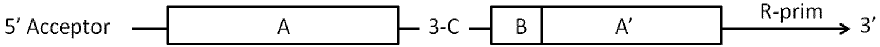 wherein:
- 5' Acceptor:: represents one member of a fluorescence resonance energy transfer (FRET) pair
- A:: represents a nucleic acid sequence motif of 10 to 30 bp
- 3-C:: represents a linker region comprised of at least three carbon atoms that prevents elongation during PCR and maintains the upstream portion of the primer, including nucleic acid sequence motif A, as single stranded at the point of cleavage by a restriction enzyme that recognizes a double stranded site
- B:: represents a single stranded motif that forms the double stranded restriction enzyme recognition site during PCR;
- A':: represents a nucleic acid sequence motif of 10 to 30 bp being complementary to the nucleic acid sequence motif of A
- R-prim:: represents a nucleic acid sequence complementary to a target sequence in a nucleic acid sequence to be amplified.

The present inventors surprisingly discovered that the use of the above primer provides:
- by covalently attaching different members of a fluorescence resonance energy transfer (FRET) pair at the 5' part of the present primer and based on the availability of FRET combinations, the simultaneous detection of 4 to 6 amplicons per reaction
- by replacing the A and A' sequence motifs as well as corresponding donor oligonucleotide sequence with sequence motifs differing approximately 5°C in melting temperature the simultaneous detection of 5 to 7 sets of sequences;
- by combining different R-prim labels and sequence motif melting temperatures the simultaneous amplification and detection of up to 40 amplicons per reaction.

The present invention uses Forster resonance energy transfer (FRET) pairs for detection. Forster resonance energy transfer (FRET), also designated as fluorescence resonance energy transfer (FRET), resonance energy transfer (RET) or electronic energy transfer (EET), is a mechanism based on energy transfer between two chromophores. A donor chromophore, initially in its electronic excited state, may transfer energy to an acceptor chromophore through non-radiative dipole-dipole coupling. The efficiency of this energy transfer is inversely proportional to the sixth power of the distance between donor and acceptor, making FRET extremely sensitive to small changes in distance. Measurements of FRET efficiency can be used to determine if two fluorophores are within a certain distance of each other. Below some fluorophores suitable to be used according to the present invention are provided:

| Fluorophore | Alternative Fluorophore | Excitation (nm) | Emission (nm) |
|---|---|---|---|
| FAM | | 495 | 515 |
| TET | CAL Fluor Gold 540 ^{A} | 525 | 540 |
| HEX | JOE, VIC ^{B}, CAL Fluor Orange 560 ^{A} | 535 | 555 |
| Cy3 ^{C} | NED ^{B}, Quasar 570 ^{A}, Oyster 556^{D} | 550 | 570 |
| TMR | CAL Fluor Red 590 ^{A} | 555 | 575 |
| ROX | LC red 610 ^{E}, CAL Fluor Red 610 ^{A} | 575 | 605 |
| Texas red | LC red 610 ^{E}, CAL Fluor Red 610 ^{A} | 585 | 605 |
| LC red 640 ^{E} | CAL Fluor Red 635 ^{A} | 625 | 640 |
| Cy5 ^{C} | LC red 670 ^{E}, Quasar 670 ^{A}, Oyster 645 ^{D} | 650 | 670 |
| LC red 705 ^{E} | ^{C} | 680 | 710 |

| Quencher | Absorption Maximum (nm) |
|---|---|
| DDQ-1^{A} | 430 |
| Dabcyl | 475 |
| Eclipse^{B} | 530 |
| Iowa Black FQ^{C} | 532 |
| BHQ-1^{D} | 534 |
| QSY-7^{E} | 571 |
| BHQ-2^{D} | 580 |
| DDQ-11^{A} | 630 |
| Iowa Black RQ^{C} | 645 |
| QSY-21^{E} | 660 |
| BHQ-3^{D} | 670 |

According to a preferred embodiment of this first aspect of the present invention, the present double stranded restriction enzyme recognition site B is a 5 to 15 bp PspG1 restriction enzyme recognition sequence. PspG1 is a restriction enzyme recognizing and cleaving the dsDNA sequence: 'CCWGG/GGWCC'. The enzyme's optimal temperature is 75°C and the enzyme is resistant to heat inactivation and, accordingly, is especially suitable to be used according to the present invention.

Another possibility is the use of other PspG1 like restriction enzymes or genome editing nuclease tools such as CRISPR, CAS9, Talen and ZFN or engineered hybrid meganucleases. Also RNase H based cleavage at the B - sequence can be envisaged by incorporating a RNA base at a specific B position of the R-prim based primer which can be cleaved, during the QPCR, by a thermostable RNase H based on the nature of RNaseH to cut at a RNA base in case of a perfect sequence similarity.

According to a second aspect, the present invention relates to Polymerase Chain Reaction (PCR) nucleic acid amplification mixtures comprising:
1) a template comprising a target sequence;
2) the present Polymerase Chain Reaction (PCR) primer;
3) a Polymerase Chain Reaction (PCR) primer S-prim allowing in combination with the present Polymerase Chain Reaction (PCR) primer amplification of at least one amplicon comprised in the target sequence;
4) a donor oligonucleotide having the following general structure 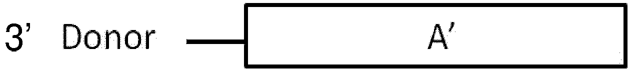 wherein
   - 3' Donor represents one member of a fluorescence resonance energy transfer (FRET) pair comprised of a light emitting molecule;
   - A' represents a nucleic acid sequence motif of 10 to 30 bp being complementary to the nucleic acid sequence motif of A;
5) restriction enzyme that recognizes the double stranded site formed during PCR, preferably PspG1;
6) amplification enzyme and nucleotides.

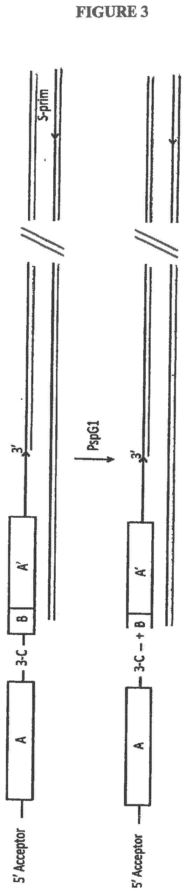

In the present amplification mixture, a detectable signal is obtained after cleavage of the restriction site B and a subsequent hybridization as is graphically depicted in **Figure 3****.**

In the present Polymerase Chain Reaction (PCR) nucleic acid amplification mixture, the present one member of a fluorescence resonance energy transfer (FRET) pair comprised of a quencher from the group consisting of Dabcyl, BHQ1, etc.

According to a third aspect, the present invention relates to the use of the present Polymerase Chain Reaction (PCR) primers or the present Polymerase Chain Reaction (PCR) nucleic acid amplification mixtures in a Polymerase Chain Reaction (PCR), preferably a quantitative Polymerase Chain Reaction (qPCR).

The present use according this aspect of the invention allows for the amplification of at least 4, preferably at least 6, more preferably at least 10 amplicons in a single nucleic acid amplification reaction such as 4, 5, 6, 7, 8, ,9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 amplicons.

The present invention will be further illustrated in the example below of an especially preferred embodiment of the present invention. In the present description, reference is made to figures wherein:
- **Figure 1:**: graphical representation of the general structure of an amplification primer according to the present invention;
- **Figure 2:**: graphical representation of the general structure of a fluorophore donor probe according to the present invention;
- **Figure 3:**: PCR and enzymatic digestion by PspG1, releasing the A sequencing motif;
- **Figure 4:**: graphical representation of signal detection using the present amplification primer and donor probe;

### Example

The RM-QPCR is short for Restriction Mediated Quantitative PCR and is developed to realize the unmet need of highly multiplexed QPCR assays. Current QPCR methods (using standard equipment) only allow limited multiplexing of up to 2 or 3 amplicons per reaction. With RM-QPCR it is theoretically possible to derive multiplexes of up to 30-40 amplicons per reaction.

### RM-QPCR:

### 1. Components:

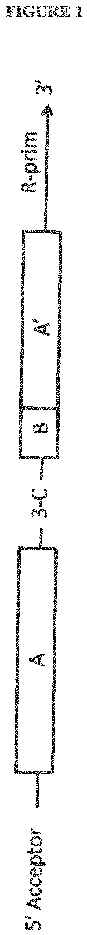

Each target amplicon is amplified by using a standard PCR primer (S-prim: 18-27 bp in length) and the present primer (R-prim: 70-80 bp in length: **Figure 1****)** composed of the following elements (from 3' to 5'):
- Amplicon specific sequence that in combination with the standard PCR primer will drive amplification if template DNA is present (R-prim).
- A' sequence motif (∼ 20 bp) which is complementary to the A sequence motif.
- B sequence motif (∼ 10 bp) containing the PspG1 restriction enzyme recognition sequence:
   ∘ PspG1 is a restriction enzyme recognising and cleaving the dsDNA sequence: 'CCWGG/GGWCC'. The enzymes optimal temperature is 75°C and the enzyme is resistant to heat inactivation and hence can be used in a PCR reaction.
- Linker region containing at least 3 C-atoms
- A sequence motif (∼ 20 bp) which is complementary to the A' sequence which contains a covalently attached fluorescent label (e.g. ROX)

### 2. Principle (simplex PCR reaction):

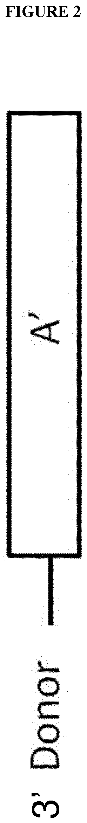

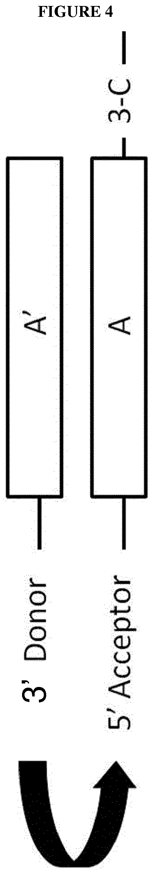

- A PCR reaction is set-up containing the following components:
   ∘ PCR buffer, dNTPs, Taq DNA polymerase
   ∘ S-prim and R-prim
   ∘ PspG1 enzyme
   ∘ Donor oligonucleotide
      ▪ The donor oligonucleotide has the sequence A' and hence is complementary to A.
      ▪ Contains a covalently attached fluorescent label (e.g. Fam or quencher)
- Upon successful amplification the single stranded B sequence motif will become double stranded leading to cleavage by PspG1 during the elongation step of the PCR (Figure 2).
- Cleavage will result in the release of the single stranded A sequence motif containing a fluorescent label. This part of the R-prim remains single stranded during PCR since elongation cannot proceed over the linker sequence as depicted in **Figure 3****.**
- The PspG1 mediated release of the A sequence motif will result in hybridisation with the donor oligonucleotide allowing detection of amplification by FRET (Fluorescence Resonance Energy Transfer) by close proximity of the fluorescent label from the A sequence motif (acceptor) and the fluorescent label on the donor oligonucleotide **(****Figure 4****)**
- Since intra-molecular hybridisation is favoured over intermolecular hybridisation, FRET will only occur if the single stranded A sequence motif is physically removed from R-prim.

### 3. Principle (multiplex PCR):

Multiplexing can be achieved by:
- Covalently attaching a different fluorescent label at the 5' of the R-prim
   ∘ Based on the availability of FRET combinations this can generate per R-prim and donor oligonucleotide 4 to 6 combinations enabling detection of 4 to 6 amplicons per reaction
- Replace A and A' sequence motif as well as corresponding donor oligonucleotide sequence with sequence motif differing ∼ 5°C in melting temperature
   ∘ Applying a ∼ 5°C melting temperature difference per set of sequence motives will result in 5 to 7 sets of sequences that can be used to detect amplicons based on melting temperature
- Combining different R-prim labels and sequence motif melting temperatures it is possible to multiplex up to ∼40 amplicons per reaction.

## Claims

1. Polymerase Chain Reaction (PCR) primer suitable for use in Restriction Mediated quantitative PCR (RM-qPCR) nucleic acid amplification reactions, said primer has the following general structure: 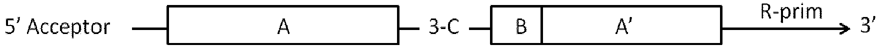 wherein:
5' Acceptor: represents one member of a fluorescence resonance energy transfer (FRET) pair
A: represents a nucleic acid sequence motif of 10 to 30 bp
3-C: represents a linker region comprised of at least three carbon atoms that prevents elongation during PCR and maintains the upstream portion of the primer, including nucleic acid sequence motif A, as single stranded at the point of cleavage by a restriction enzyme that recognizes a double stranded site
B: represents a single stranded motif that forms the double stranded restriction enzyme recognition site during PCR;
A': represents a nucleic acid sequence motif of 10 to 30 bp being complementary to the nucleic acid sequence motif of A
R-prim: represents a nucleic acid sequence complementary to a target sequence in a nucleic acid sequence to be amplified.

2. Polymerase Chain Reaction (PCR) primer according to claim 1, wherein said double stranded restriction enzyme recognition site is a 5 to 15 bp PspG1 restriction enzyme recognition sequence.

3. Polymerase Chain Reaction (PCR) primer according to claim 2, wherein said 5 to 15 bp PspG1 restriction enzyme recognition sequence comprises the sequence motif "CCWGG" or "GGWCC" wherein W is A or T.

4. Polymerase Chain Reaction (PCR) primer according to any of the claims 1 to 3, wherein said one member of a fluorescence resonance energy transfer (FRET) pair is a fluorophore selected from the group consisting of FAM, TET, HEX, Cy3, TMR, ROX, Texas red, LC red 640, Cy5, a quencher and LC red 705.

5. Polymerase Chain Reaction (PCR) nucleic acid amplification mixture comprising:
1) a template comprising a target sequence;
2) a Polymerase Chain Reaction (PCR) primer as defined in any of the claims 1 to 4;
3) a Polymerase Chain Reaction (PCR) primer S-prim providing, in combination with a Polymerase Chain Reaction (PCR) primer as defined in any of the claims 1 to 4, amplification of at least one amplicon comprised in said target sequence;
4) a donor oligonucleotide having the following general structure:
5) 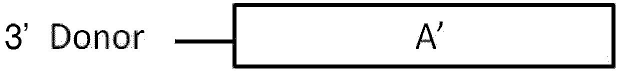 wherein
- A' represents a nucleic acid sequence motif of 10 to 30 bp being complementary to the nucleic acid sequence motif of A;
6) a restriction enzyme that recognizes the double stranded site formed during PCR, preferably PspG1;
7) amplification enzyme and nucleotides.

6. Polymerase Chain Reaction (PCR) nucleic acid amplification mixture according to claim 5, wherein said one member of a fluorescence resonance energy transfer (FRET) pair comprised of ROX is a fluorophore selected from the group consisting of FAM, TET, HEX, Cy3, TMR, ROX, Texas red, LC red 640, Cy5, quencher and LC red 705.

7. Use of a Polymerase Chain Reaction (PCR) primer according to any of the claims 1 to 4 or a Polymerase Chain Reaction (PCR) nucleic acid amplification mixture according to claim 5 or claim 6 in a Polymerase Chain Reaction (PCR).

8. Use according to claim 7, wherein said Polymerase Chain Reaction (PCR) is a quantitative Polymerase Chain Reaction (qPCR).

9. Use according to claim 7 or claim 8 wherein said Polymerase Chain Reaction (PCR) or said quantitative Polymerase Chain Reaction (qPCR) provides amplification of at least 4 amplicons in a single nucleic acid amplification reaction.

10. Use according to claim 7 or claim 8 wherein said Polymerase Chain Reaction (PCR) or said quantitative Polymerase Chain Reaction (qPCR) provides amplification of at least 6 amplicons in a single nucleic acid amplification reaction.

11. Use according to claim 7 or claim 8 wherein said Polymerase Chain Reaction (PCR) or said quantitative Polymerase Chain Reaction (qPCR) provides amplification of at least 10 amplicons in a single nucleic acid amplification reaction.

## Patentansprüche

1. Polymerasekettenreaktions(PCR)-Primer, der zur Verwendung in restriktionsvermittelten quantitativen PCR (RM-qPCR)-Nukleinsäure-Amplifikationsreaktionen geeignet ist, wobei der Primer die folgende allgemeine Struktur aufweist 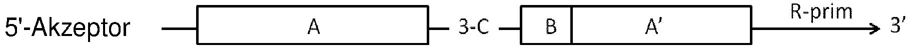 wobei:
5'-Akzeptor: ein Element eines Fluoreszenz-Resonanz-Energie-Transfer (FRET)-Paares darstellt
A: ein Nukleinsäuresequenzmotiv von 10 bis 30 bp darstellt
3-C: eine Linkerregion darstellt, die aus mindestens drei Kohlenstoffatomen besteht, die die Elongation während der PCR verhindert und den stromaufwärts gelegenen Abschnitt des Primers, einschließlich Nukleinsäuresequenzmotiv A, als einzelsträngig an dem Punkt der Spaltung durch ein Restriktionsenzym, das eine doppelsträngige Stelle erkennt, beibehält
B: ein Einzelstrangmotiv darstellt, das die Doppelstrang-Restriktionsenzym-Erkennungsstelle während der PCR bildet;
A': ein Nukleinsäuresequenzmotiv von 10 bis 30 bp darstellt, das komplementär zu dem Nukleinsäuresequenzmotiv von A ist
R-prim: eine Nukleinsäuresequenz darstellt, die zu einer Zielsequenz in einer zu amplifizierenden Nukleinsäuresequenz komplementär ist.

2. Polymerasekettenreaktions(PCR)-Primer nach Anspruch 1, wobei die Doppelstrang-Restriktionsenzym-Erkennungsstelle eine 5 bis 15 bp PspG1-Restriktionsenzym-Erkennungssequenz ist.

3. Polymerasekettenreaktions(PCR)-Primer nach Anspruch 2, wobei die 5 bis 15 bp PspG1-Restriktionsenzym-Erkennungssequenz das Sequenzmotiv "CCWGG" oder "GGWCC" umfasst, wobei W A oder T ist.

4. Polymerasekettenreaktions(PCR)-Primer nach einem der Ansprüche 1 bis 3, wobei das eine Element eines Fluoreszenz-Resonanz-Energietransfer (FRET)-Paares ein Fluorophor ist, das aus der Gruppe ausgewählt ist, bestehend aus FAM, TET, HEX, Cy3, TMR, ROX, Texas Red, LC Red 640, Cy5, einem Quencher und LC Red 705.

5. Polymerasekettenreaktions(PCR)-Nukleinsäureamplifikationsmischung umfassend:
1) ein Templat, das eine Zielsequenz umfasst;
2) einen Polymerasekettenreaktionsprimer (PCR-Primer) nach einem der Ansprüche 1 bis 4;
3) einen Polymerasekettenreaktions(PCR)-Primer S-prim, der, in Kombination mit einem Polymerasekettenreaktions(PCR)-Primer nach einem der Ansprüche 1 bis 4, die Amplifikation von mindestens einem Amplicon, das in der Zielsequenz mit umfasst ist, bereitstellt;
4) ein Donor-Oligonukleotid mit der folgenden allgemeinen Struktur:
5) 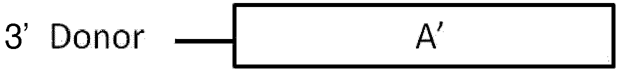 wobei
- A' ein Nukleinsäuresequenzmotiv von 10 bis 30 bp darstellt, das komplementär zu dem Nukleinsäuresequenzmotiv von A ist;
6) ein Restriktionsenzym, das die während der PCR gebildete Doppelstrangstelle erkennt, vorzugsweise PspGI;
7) Amplifizierungsenzym und Nukleotide.

6. Polymerasekettenreaktions(PCR)-Nukleinsäureamplifikationsmischung nach Anspruch 5, wobei das eine Element eines Fluoreszenz-Resonanz-Energie-Transfer (FRET)-Paares, das ROX umfasst, ein Fluorophor ist, das aus der Gruppe ausgewählt ist bestehend aus FAM, TET, HEX, Cy3, TMR, ROX, Texas Red, LC Red 640, Cy5, Quencher und LC Red 705.

7. Verwendung eines Polymerasekettenreaktions(PCR)-Primers nach einem der Ansprüche 1 bis 4 oder einer Polymerasekettenreaktions(PCR)-Nukleinsäureamplifikationsmischung nach Anspruch 5 oder 6 bei einer Polymerasekettenreaktion (PCR).

8. Verwendung nach Anspruch 7, wobei die Polymerasekettenreaktion (PCR) eine quantitative Polymerasekettenreaktion (qPCR) ist.

9. Verwendung nach Anspruch 7 oder 8, wobei die Polymerasekettenreaktion (PCR) oder die quantitative Polymerasekettenreaktion (qPCR) Amplifikation von mindestens 4 Amplicons in einer einzelnen Nukleinsäure-Amplifikationsreaktion bereitstellt.

10. Verwendung nach Anspruch 7 oder Anspruch 8, wobei die Polymerasekettenreaktion (PCR) oder die quantitative Polymerasekettenreaktion (qPCR) Amplifikation von mindestens 6 Amplicons in einer einzelnen Nukleinsäure-Amplifikationsreaktion bereitstellt.

11. Verwendung nach Anspruch 7 oder 8, wobei die Polymerasekettenreaktion (PCR) oder die quantitative Polymerasekettenreaktion (qPCR) Amplifikation von mindestens 10 Amplicons in einer einzelnen Nukleinsäure-Amplifikationsreaktion bereitstellt.

## Revendications

1. Amorce de réaction en chaîne par polymérase (PCR) appropriée pour une utilisation dans des réactions d'amplification d'acide nucléique par PCR quantitative médiée par restriction (RM-qPCR), ladite amorce a la structure générale suivante : 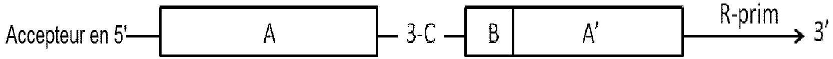 où :
Accepteur en 5' : représente un élément d'une paire de transfert d'énergie par résonance de fluorescence (FRET)
A : représente un motif de séquence d'acide nucléique de 10 à 30 pb
3-C : représente une région de liaison constituée d'au moins trois atomes de carbone qui empêche l'élongation pendant la PCR et maintient la partie amont de l'amorce, comportant le motif de séquence d'acide nucléique A, comme motif simple brin au point de clivage par une enzyme de restriction qui reconnaît un site double brin
B : représente un motif simple brin qui forme le site de reconnaissance d'enzyme de restriction double brin pendant la PCR ;
A' : représente un motif de séquence d'acide nucléique de 10 à 30 pb qui est complémentaire du motif de séquence d'acide nucléique de A
R-prim : représente une séquence d'acide nucléique complémentaire d'une séquence cible dans une séquence d'acide nucléique à amplifier.

2. Amorce de réaction en chaîne par polymérase (PCR) selon la revendication 1, où ledit site de reconnaissance d'enzyme de restriction double brin est une séquence de reconnaissance d'enzyme de restriction PspG1 de 5 à 15 pb.

3. Amorce de réaction en chaîne par polymérase (PCR) selon la revendication 2, où ladite séquence de reconnaissance d'enzyme de restriction PspG1 de 5 à 15 pb comprend le motif de séquence "CCWGG" ou "GGWCC" où W représente A ou T.

4. Amorce de réaction en chaîne par polymérase (PCR) selon l'une des revendications 1 à 3, où ledit élément d'une paire de transfert d'énergie par résonance de fluorescence (FRET) est un fluorophore choisi dans le groupe consistant en FAM, TET, HEX, Cy3, TMR, ROX, Texas red, LC red 640, Cy5, un désactiveur et LC red 705.

5. Mélange d'amplification d'acide nucléique par réaction en chaîne par polymérase (PCR) comprenant :
1) une matrice comprenant une séquence cible ;
2) une amorce de réaction en chaîne par polymérase (PCR) telle que définie dans l'une des revendications 1 à 4 ;
3) une amorce S-prim de réaction en chaîne par polymérase (PCR) fournissant, en combinaison avec une amorce de réaction en chaîne par polymérase (PCR) telle que définie dans l'une des revendications 1 à 4, une amplification d'au moins un amplicon compris dans ladite séquence cible ;
4) un oligonucléotide donneur ayant la structure générale suivante :
5) 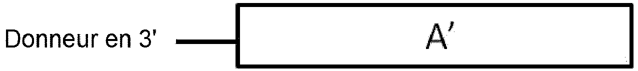 où
- A' : représente un motif de séquence d'acide nucléique de 10 à 30 pb qui est complémentaire du motif de séquence d'acide nucléique de A ;
6) une enzyme de restriction qui reconnaît le site double brin formé pendant la PCR, de préférence PspG1 ;
7) une enzyme d'amplification et des nucléotides.

6. Mélange d'amplification d'acide nucléique par réaction en chaîne par polymérase (PCR) selon la revendication 5, où ledit élément d'une paire de transfert d'énergie par résonance de fluorescence (FRET) constitué de ROX est un fluorophore choisi dans le groupe consistant en FAM, TET, HEX, Cy3, TMR, ROX, Texas red, LC red 640, Cy5, un désactiveur et LC red 705.

7. Utilisation d'une amorce de réaction en chaîne par polymérase (PCR) selon l'une des revendications 1 à 4 ou d'un mélange d'amplification d'acide nucléique par réaction en chaîne par polymérase (PCR) selon la revendication 5 ou 6 dans une réaction en chaîne par polymérase (PCR).

8. Utilisation selon la revendication 7, où ladite réaction en chaîne par polymérase (PCR) est une réaction en chaîne par polymérase quantitative (qPCR).

9. Utilisation selon la revendication 7 ou la revendication 8, où ladite réaction en chaîne par polymérase (PCR) ou ladite réaction en chaîne par polymérase quantitative (qPCR) fournit une amplification d'au moins 4 amplicons dans une seule réaction d'amplification d'acide nucléique.

10. Utilisation selon la revendication 7 ou la revendication 8, où ladite réaction en chaîne par polymérase (PCR) ou ladite réaction en chaîne par polymérase quantitative (qPCR) fournit une amplification d'au moins 6 amplicons dans une seule réaction d'amplification d'acide nucléique.

11. Utilisation selon la revendication 7 ou la revendication 8, où ladite réaction en chaîne par polymérase (PCR) ou ladite réaction en chaîne par polymérase quantitative (qPCR) fournit une amplification d'au moins 10 amplicons dans une seule réaction d'amplification d'acide nucléique.
